(19) Europäisches Patentamt
European Patent Office
Office européen des brevets

(11) Veröffentlichungsnummer : **0 101 974**
**B1**

(12) # EUROPÄISCHE PATENTSCHRIFT

(45) Veröffentlichungstag der Patentschrift : **02.01.86**

(51) Int. Cl.[4] : **C 07 D 285/16**, A 01 N 43/88

(21) Anmeldenummer : **83107684.9**

(22) Anmeldetag : **04.08.83**

(54) **Thiadiazinone, Verfahren zu ihrer Herstellung und diese enthaltende Fungizide.**

(30) Priorität : **20.08.82 DE 3230923**

(43) Veröffentlichungstag der Anmeldung : **07.03.84 Patentblatt 84/10**

(45) Bekanntmachung des Hinweises auf die Patenterteilung : **02.01.86 Patentblatt 86/01**

(84) Benannte Vertragsstaaten : **AT BE CH DE FR GB IT LI NL SE**

(56) Entgegenhaltungen :
**CENTRAL PATENTS INDEX BASIC ABSTRACTS JOURNAL, Section C: AGDOC, B08, 18. April 1979, Seite 35, Nr. 14890B/08, Derwent Publications Ltd., London, GB.**

(73) Patentinhaber : **BASF Aktiengesellschaft**
**Carl-Bosch-Strasse 38**
**D-6700 Ludwigshafen (DE)**

(72) Erfinder : **Acker, Rolf-Dieter, Dr.**
**Tuchbleiche 8**
**D-6906 Leimen (DE)**
Erfinder : **Ammermann, Eberhard, Dr.**
**Sachsenstrasse 3**
**D-6700 Ludwigshafen (DE)**
Erfinder : **Pommer, Ernst-Heinrich, Dr.**
**Berliner Platz 7**
**D-6703 Limburgerhof (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

EP 0 101 974 B1

**Beschreibung**

Die vorliegende Erfindung betrifft Thiadiazinone, Verfahren zu ihrer Herstellung, Fungizide, die diese Verbindungen als Wirkstoffe enthalten, sowie ein Verfahren zur Bekämpfung unerwünschten Pilzbefalls mit diesen Wirkstoffen.

Thiadiazinone, z. B. das 2,6-Dimethyl-4-(N-ethylcarbamoyl)-3,5dioxo-2H-3,4,5,6-tetrahydro-1,2,6-thiadiazin-1,1-dioxid, sind als Insektizide und Miticide bekannt (Japan. Offenlegungsschrift J 54005-991). Es ist ferner bekannt, N-Trichlormethyl-thiotetrahydrophthalimid als Fungizid zu verwenden (Chemical Week, 21. Juni 1972, Seite 46).

Aus Central Patents Index Basic Abstracts Journal, Section C AGDOC, B08, 18. April 1979, Seite 35, Nr. 14890B/08 Derwent Publications, Londonc GB sind Thiadiazinone bekannt, die durch einen Halogenphenylcarbamoylrest substituiert sind. Im Gegensatz dazu enthalten die erfindungsgemäßen Verbindungen keinen Halogenphenylcarbamoylrest.

Es wurde nun gefunden, daß Thiadiazinone der Formel

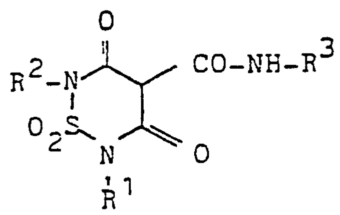

(I)

in der

$R^1$ und $R^2$ gleich oder verschieden sind und Wasserstoff, $C_1$-$C_{10}$-Alkyl, $C_3$-$C_8$-Cycloalkyl, Phenyl oder durch Halogen, Alkoxy, Alkyl oder Halogenalkoxy substituiertes Phenyl und $R^3$ Phenyl, halogenalkylsubstituiertes, alkylsubstituiertes, alkoxysubstituiertes, halogenalkoxysubstituiertes, oder gleichzeitig durch Alkyl und Halogen substituiertes Phenyl bedeuten,

eine gute fungizide Wirkung besitzen.

Aufgrund der Keto-Enol-Tautomerie können Verbindungen der Formel I auch in den Formen

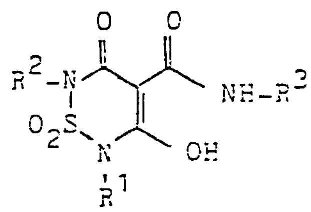

(Ia)

und

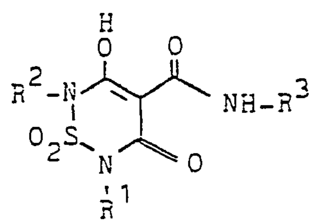

(Ib)

auftreten. Hier ist auch die Bildung von Salzen möglich.

Die vorliegende Erfindung umfaßt sowohl die Verbindungen I, Ia und Ib als auch ihre Mischungen und ihre Salze (Natriumsalze, Kaliumsalze).

$C_1$-$C_{10}$-Alkylreste sind beispielsweise Methyl, Ethyl, n-Propyl, Isopropyl, n-Butyl, Isobutyl, sek.-Butyl, n-Pentyl, Isooctyl, n-Decyl ; $C_3$-$C_8$-Cycloalkylreste sind beispielsweise Cyclopropyl, Cyclobutyl, Cyclopentyl, Cyclohexyl, Cyclooctyl, Phenylreste, substituiert durch Halogen (sind beispielsweise substituiert durch Fluor, Chlor, Brom, Iod) oder durch Alkoxy (Methoxy), Alkyl (Methyl), Halogenalkoxy (Trifluormethoxy), halogenalkylsubstituiertes Phenyl ist Phenyl, beispielsweise substituiert durch Halogen-(Fluor, Chlor, Brom)alkyl mit 1 bis 4 C-Atomen, z. B. Chlormethyl, Chlorethyl, Trifluormethyl, Pentafluorethyl, alkylsubstituiertes Phenyl ist Phenyl, beispielsweise substituiert durch Alkyl mit 1 bis 4 C-Atomen, z. B. Methyl, Ethyl, Isopropyl, prim., sek., tert.-Butyl, alkoxysubstituiertes Phenyl ist Phenyl, beispielsweise substituiert durch Alkoxy mit 1 bis 4 C-Atomen, z. B. Methoxy, Ethoxy, Isopropoxy, t-Butoxy, halogenalkoxysubstituiertes Phenyl ist Phenyl, beispielsweise substituiert durch Halogen-(Fluor, Chlor, Brom)-alkoxy mit 1 bis 4 C-Atomen, z. B. Trifluormethoxy, Pentafluorethoxy,

Gleichzeitig durch Alkyl und Halogen substituiertes Phenyl ist Phenyl, beispielsweise gleichzeitig

substituiert durch Halogen (Fluor, Chlor, Brom) und Alkyl mit 1 bis 4 C-Atomen, z. B. 2-Chlor-4-methylphenyl, 4-Fluor-3-ethylphenyl, 4-Brom-2-methylphenyl.

Man erhält die neuen Thiadiazinone der Formel I, wenn man Thiadiazinone der Formel

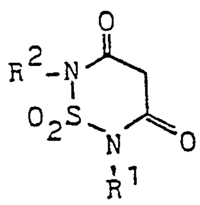

(II)

in der $R^1$ und $R^2$ die vorgenannten Bedeutungen besitzen, in einem inerten Lösungsmittel mit einem Isocyanat der Formel

$$R^3—NCO \quad (III)$$

gegebenenfalls in Gegenwart einer Base umsetzt.

Die Umsetzung wird beispielsweise bei einer Temperatur von 25 bis 200 °C, vorzugsweise 50 und 150 °C durchgeführt. Die Reaktionszeit beträgt beispielsweise zwischen 0,5 und 12 Stunden. Die Komponenten II und III werden beispielsweise in Molverhältnissen von 1 : 1 bis zu 1 : 10 (II : III) eingesetzt ; zur Beschleunigung der Reaktion kann eine Base im Molverhältnis 0,1 bis 1,0, bezogen auf 1 Mol der Verbindung der Formel II, zugesetzt werden.

Als basische Katalysatoren kommen tertiäre Amine, Erdalkaliverbindungen, Ammoniumverbindungen und Alkaliverbindungen sowie entsprechende Gemische in Betracht. Auch Zinkverbindungen können verwendet werden. Beispiele hierfür sind : Kaliumhydroxid, Natriumhydroxid, Kaliumcarbonat, Natriumcarbonat, Lithiumhydroxid, Lithiumcarbonat, Natriumhydrogencarbonat, Kaliumhydrogencarbonat, Calciumhydroxid, Calciumoxid, Bariumoxid, Magnesiumhydroxid, Magnesiumoxid, Bariumhydroxid, Calciumcarbonat, Magnesiumcarbonat, Magnesiumhydrogencarbonat, Magnesiumacetat, Zinkhydroxid, Zinkoxid, Zinkcarbonat, Zinkacetat, Natriumformiat, Natriumacetat, Trimethylamin, Triethylamin, Tripropylamin, Triisopropylamin, Tributylamin, Triisobutylamin, Tri-sec-butylamin, Tri-tert.-butylamin, Tribenzylamin, Tricyclohexylamin, Triamylamin, Diisopropylethylamin, Trihexylamin, N,N-Dimethylanilin, N,N-Diethylanilin, N,N-Dipropylanilin, N,N-dimethyltoluidin, N,N-diethyltoluidin, N,N-dipropyltoluidin, N,N-Dimethyl-p-aminopyridin, N,N-Diethyl-p-aminopyridin, N,N-Dipropyl-p-aminopyridin, N-Methylpyrrolidon, N-Ethylpyrrolidon, N-Methylpiperidin, N-Ethylpiperidin, N-Methylpyrrolidin, N-Ethylpyrrolidin, N-Methylimidazol, N-Ethylimidazol, N-Methylpyrrol, N-Ethylpyrrol, N-Methylmorpholin, N-Ethylmorpholin, N-Methylhexamethylenimin, N-Ethylhexamethylenimin, Pyridin, Chinolin, alpha-Picolin, beta-Picolin, gamma-Picolin, Isochinolin, Pyrimidin, Acridin, N,N,N',N'-Tetramethylethylendiamin, N,N,N',N'-Tetraethylethylendiamin, Chinoxalin, Chinazolin, N-Propyldiisopropylamin, N,N'-Dimethylcyclohexylamin, 2,6-Lutidin, 2,4-Lutidin, Trifurylamin, Triethylendiamin.

Außer den vorgenannten anorganischen Basen kommen außerdem z. B. Natriumpropionat, Natriumbutyrat, Natriumisobutyrat, Kaliumformiat, Kaliumacetat, Kaliumpropionat, Kaliumbutyrat, Kaliumisobutyrat, Natriummethylat, Natriumethylat, Natriumpropylat, Natriumisopropylat, Natriumbutylat, Natriumisobutylat, Natrium-sec-butylat, Natrium-tert.-butylat, Natriumethylenglykolat, Natriumpropylen-(1,2)-glykolat, Natriumpropylen-(1,3)-glykolat, Natriumdiethylenglykolat, Natriumtriethylenglykolat, Natriumdipropylen-(1,2)-glykolat, Kaliummethylat, Kaliumethylat, Kalium-n-propylat, Kaliumisopropylat, Kalium-n-butylat, Kalium-isobutylat, Kalium-sec-butylat, Kalium-tert.-butylat, Kaliummethylenglykolat, Kaliumpropylen-(1,2)-glykolat, Kaliumpropylen-(1,3)-glykolat, Kaliumdiethylenglykolat, Kaliumtriethylenglykolat, Kaliumdipropylen-(1,2)-glykolat in Betracht.

Als Lösungsmittel kommen z. B. Halogenkohlenwasserstoffe, insbesondere Chlorkohlenwasserstoffe, z. B. Tetrachlorethylen, 1,1,2,2- oder 1,1,1,2-Tetrachlorethan, Dichlorpropan, Methylenchlorid, Dichlorbutan, Chloroform, Chlornaphthalin, Dichlornaphthalin, Tetrachlorkohlenstoff, 1,1,1- oder 1,1,2-Trichlorethan, Trichlorethylen, Pentachlorethan, o-, m-, p-Difluorbenzol, 1,2-Dichlorethan, 1,1-Dichlorethan, 1,2-cis-Dichlorethylen, Chlorbenzol, Fluorbenzol, Brombenzol, Jodbenzol, o-, p- und m-Dichlorbenzol, o- p-, m-Dibrombenzol, o-, m-, p-Chlortoluol, 1,2,4-Trichlorbenzol ; Ether, z. B. Ethylpropylether, Methyl-tert.-butylether, n-Butylethylether, Di-n-butylether, Di-isobutylether, Diisoamylether, Diisopropylether, Anisol, Phenetol, Cyclohexylmethylether, Diethylether, Ethylenglykoldimethylether, Tetrahydrofuran, Dioxan, Thioanisol, beta, beta'-Dichlordiethylether ; Nitrokohlenwasserstoffe, wie Nitromethan, Nitroethan, Nitrobenzol, o-, m-, p-Chlornitrobenzol, o-Nitrotoluol ; Nitrile, wie Acetonitril, Butyronitril, Isobutyronitril, Benzonitril, m-Chlorbenzonitril ; aliphatische, cycloaliphatische oder aromatische Kohlenwasserstoffe, z. B. Heptan, Pinan, Nonan, o-, m-, p-Cymol, Benzinfraktionen innerhalb eines Siedepunktintervalls von 70 bis 190 °C, Cyclohexan, Methylcyclohexan, Dekalin, Petrolether, Hexan, Ligroin, 2,2,4-Trimethylpentan, 2,2,3-Trimethylpentan, 2,3,3-Trimethylpentan, Octan, Toluol, o-, m-, p-Xylol, Tetralin ; Ester, z. B. Ethylacetat, Acetessigester, Isobutylacetat ; Amide, z. B. Formamid, Methylformamid, Dimethylformamid ; Ketone, z. B. Aceton, Methylethylketon ; Sulfoxide wie Dimethylsulfoxid, Heteroaromaten wie Pyridin, alpha-, beta-, gamma-Picolin, Pyrimidin ; Wasser oder Alkohole wie Methanol, Ethanol und Isopropanol ; und entsprechende Gemische in Betracht. Zweckmäßig verwendet man das Lösungsmittel in einer Menge von 100 bis 2 000 Gew.%, vorzugsweise von 200 bis 700 Gew.%, bezogen auf Ausgangsstoff II.

Das Verfahren kann kontinuierlich oder diskontinuierlich, drucklos oder unter Druck, durchgeführt werden ; der Einfachheit halber wird Atmosphärendruck bevorzugt.

Die Abtrennung der Verbindungen der Formel I und die Beendigung der Reaktion kann beispielsweise wie folgt durchgeführt werden : Entweder werden die flüchtigen Anteile des Reaktionsgemisches im Vakuum abdestilliert und die im Rückstand verbleibenden Rohprodukte der Formel I umkristallisiert oder gegebenenfalls chromatographiert oder man gießt die Reaktionsmischung in eine eventuell gekühlte oder verdünnte wäßrige Lösung einer Mineralsäure, z. B. Salzsäure oder Schwefelsäure (diese Art der Aufarbeitung wird bevorzugt bei Verwendung größerer Mengen Base bei der Umsetzung) und kristallisiert den entstehenden Niederschlag um oder trennt die organische Phase ab und engt sie danach ein. Die weitere Reinigung kann in üblicher Weise z. B. durch Umkristallisieren oder Chromatographie erfolgen.

Vorschrift 1

38,9 Teile (Gewichtsteile) Methylamidosulfonylchlorid wurden zu einer Mischung von 17,7 Teilen Isopropylamin, 33,3 Teilen Triethylamin und 120 Teilen Tetrahydrofuran (THF), die zwischen − 70 und − 65 °C gehalten wurde, portionsweise zugefügt. Nach 3 Stunden Rühren bei 20 °C wurde vom Ungelösten abfiltriert und die Lösung zur Trockene eingeengt. Der Filtratrückstand wurde mit Methylenchlorid ausgerührt, filtriert und die Lösung eingeengt. Man erhielt 30,6 Teile N-Methyl-N′-isopropylsulfamid, Fp. 50 bis 52 °C (Ausgangsprodukt).

Vorschrift 2

29,5 Teile Isopropylamin und 55,6 Teile Triethylamin werden in 320 Teilen THF vorgelegt. Zwischen 0 und −5 °C fügt man 60 Teile Trimethylchlorsilan portionsweise unter Stickstoff zu und rührt 30 Minuten bei Raumtemperatur und 1 Stunde bei 60 °C nach. Man kühlt auf − 10 °C ab und gibt portionsweise 33,8 Teile Sulfurylchlorid zu, läßt die Mischung auf Raumtemperatur erwärmen und rührt 2 Stunden nach. Trimethylchlorsilan und das Lösungsmittel werden abdestilliert und der Rückstand mit Eiswasser gewaschen. Man erhält 30,5 Teile N,N′-Diisopropylsulfamid, Fp. 98 bis 101 °C (Ausgangsprodukt).

Vorschrift 3

22 Teile N-Methyl-N′-isopropylsulfamid und 20,4 Teile Malonsäurechlorid werden in 350 Teilen Toluol 30 Min. bei 50 °C und 14 Stunden bei 70 °C gerührt. Nach dem Abkühlen wird die Lösung vom Öl abdekantiert und aus der Lösung das Lösungsmittel abdestilliert. Man erhält 24 Teile 2-Methyl-6-isopropyl-3,5-dioxo-2H-3,4,5,6-tetrahydro-1,2,6-thiadiazin-1,1-dioxid, $n_D^{22} = 1{,}490\,6$ (Ausgangsprodukt).

Beispiel 1

8,8 Teile (Gewichtsteile) 2-Methyl-6-isopropyl-3,5-dioxo-2H-3,4,5,6-tetrahydro-1,2,6-thiadiazin-1,1-dioxid, 6,0 Teile Phenylisocyanat und 80 Teile Pyridin wurden 30 Min. bei 22 °C und 3 Stunden bei Rückfluß gerührt. Nach dem Abkühlen rührte man die Lösung in Eiswasser/Salzsäure ein und schüttelte die Mischung mit Methylenchlorid zweimal aus. Die organische Phase wurde getrocknet, eingeengt und der Rückstand aus Ethanol umkristallisiert. Es wurden 13 Teile 2-Methyl-4-(N-phenylcarbamoyl)-6-isopropyl-3,5-dioxo-2H-3,4,5,6-tetrahydro-1,2,6-thiadiazin-1,1-dioxid, Fp. 118 bis 120 °C, erhalten (Verbindung Nr. 7).

Beispiel 2

10 Teile Isobutyl-3,5-dioxo-2H-3,4,5,6-tetrahydro-1,2,6-thiadiazin-1,1-dioxid, 5,1 Teile Phenylisocyanat und 70 Teile Pyridin wurden 3 Stunden bei Rückfluß gerührt. Nach dem Einrühren in Eiswasser/Salzsäure (Mischung wurde auf pH 2 gebracht) ließ man 30 Min. stehen, saugte den Rückstand ab und kristallisierte ihn in Ethanol um. Es wurden 4,5 Teile 2 Isobutyl-4-(N-phenylcarbamoyl)-6H-3,5-dioxo-2H-3,4,5,6-tetrahydro-1,2,6-thiadiazin-1,1,-dioxid, Fp. 82 bis 84 °C, erhalten (Verbindung Nr. 17).

Beispiel 3

7,3 Teile 2,6-Dimethyl-3,5-dioxo-2H-3,4,5,6-tetrahydro-1,2,6-thiadiazin-1,1-dioxid, 6,8 Teile Phenylisocyanat, 1,6 Teile Triethylamin und 65 Teile Toluol wurden 7 Stunden unter Rückfluß gerührt. Nach dem Abkühlen und Abdestillieren des Lösungsmittels verblieb ein kristalliner Rückstand, der in Ethanol umkristallisiert wurde. Man erhielt 9,1 Teile 2,6-Dimethyl-4-(N-phenylcarbamoyl)-3,5-dioxo-2H-3,4,5,6-tetrahydro-1,2,6-thiadiazin-1,1-dioxid, Fp. 151 bis 153 °C (Verbindung Nr. 1).

Entsprechend wurden diejenigen in der folgenden Tabelle angegebenen Verbindungen der Formel I hergestellt, deren Schmelzpunkte (Fp) angegeben sind. Ihre Struktur wurde durch Ultrarot- und Kernresonanz-Spektroskopie sowie durch Elementaranalyse gesichert. Die Verbindungen, bei denen keine Angaben zur physikalischen Chemie gemacht sind, können auf die gleiche Art und Weise wie bei den tatsächlich hergestellten Verbindungen erhalten werden ; es kann erwartet werden, daß sie aufgrund ihrer ähnlichen Konstitution ähnliche Wirkungen wie die näher untersuchten Verbindungen haben.

| Nr. | $R^1$ | $R^2$ | $R^3$ | | Fp. [°C] |
|---|---|---|---|---|---|
| 1 | $CH_3$ | $CH_3$ | $C_6H_5$ | | 151-153 |
| 2 | $CH_3$ | $C_2H_5$ | $C_6H_5$ | | |
| 3 | $C_2H_5$ | $C_2H_5$ | $C_6H_5$ | | 116-118 |
| 4 | $CH_3$ | $n-C_3H_7$ | $C_6H_5$ | | 114-116 |
| 5 | $C_2H_5$ | $n-C_3H_7$ | $C_6H_5$ | | |
| 6 | $n-C_3H_7$ | $n-C_3H_7$ | $C_6H_5$ | | 72- 74 |
| 7 | $CH_3$ | $i-C_3H_7$ | $C_6H_5$ | | 118-120 |
| 8 | $C_2H_5$ | $i-C_3H_7$ | $C_6H_5$ | | |
| 9 | $n-C_3H_7$ | $i-C_3H_7$ | $C_6H_5$ | | |
| 10 | $i-C_3H_7$ | $i-C_3H_7$ | $C_6H_5$ | | 82- 84 |
| 11 | $i-C_3H_7$ | $i-C_3H_7$ | $C_6H_5$ | (Na-Salz) | |
| 12 | $CH_3$ | $n-C_4H_9$ | $C_6H_5$ | | 120-123 |
| 13 | $CH_3$ | $n-C_4H_9$ | $C_6H_5$ | (Na-Salz) | 235 |
| 14 | $C_2H_5$ | $n-C_4H_9$ | $C_6H_5$ | | |
| 15 | $i-C_3H_7$ | $n-C_4H_9$ | $C_6H_5$ | | |
| 16 | $n-C_4H_9$ | $n-C_4H_9$ | $C_6H_5$ | | |
| 17 | H | $i-C_4H_9$ | $C_6H_5$ | | 82- 84 |
| 18 | $CH_3$ | $i-C_4H_9$ | $C_6H_5$ | | |
| 19 | $C_2H_5$ | $i-C_4H_9$ | $C_6H_5$ | | |
| 20 | $n-C_3H_7$ | $i-C_4H_9$ | $C_6H_5$ | | |
| 21 | $i-C_3H_7$ | $i-C_4H_9$ | $C_6H_5$ | | |
| 22 | $n-C_4H_9$ | $i-C_4H_9$ | $C_6H_5$ | | |
| 23 | $i-C_4H_9$ | $i-C_4H_9$ | $C_6H_5$ | | |
| 24 | $CH_3$ | $t-C_4H_9$ | $C_6H_5$ | | |
| 25 | $CH_3$ | Cyclopropyl | $C_6H_5$ | | |
| 26 | $i-C_3H_7$ | Cyclopropyl | $C_6H_5$ | | |
| 27 | $CH_3$ | Cyclobutyl | $C_6H_5$ | | |
| 28 | $i-C_3H_7$ | Cyclobutyl | $C_6H_5$ | | |
| 29 | $CH_3$ | Cyclopentyl | $C_6H_5$ | | |
| 30 | $n-C_3H_7$ | Cyclopentyl | $C_6H_5$ | | |
| 31 | $i-C_3H_7$ | Cyclopentyl | $C_6H_5$ | | |
| 32 | Cyclopentyl | Cyclopentyl | $C_6H_5$ | | |
| 33 | Cyclobutyl | Cyclobutyl | $C_6H_5$ | | |
| 34 | Cyclopropyl | Cyclopropyl | $C_6H_5$ | | |
| 35 | $CH_3$ | Cyclohexyl | $C_6H_5$ | | |
| 36 | $C_2H_5$ | Cyclohexyl | $C_6H_5$ | | |
| 37 | $i-C_3H_7$ | Cyclohexyl | $C_6H_5$ | | |

Fortsetzung

| Nr. | $R^1$ | $R^2$ | $R^3$ | Fp.[°C] |
|---|---|---|---|---|
| 38 | Cyclohexyl | Cyclohexyl | $C_6H_5$ | 103-106 |
| 39 | Cyclohexyl | Cyclohexyl | $C_6H_5$ (Na-Salz) | 240 |
| 40 | $CH_3$ | $C_6H_5$ | $C_6H_5$ | |
| 41 | $C_2H_5$ | $C_6H_5$ | $C_6H_5$ | |
| 42 | $n-C_3H_7$ | $C_6H_5$ | $C_6H_5$ | |
| 43 | $i-C_3H_7$ | $C_6H_5$ | $C_6H_5$ | |
| 44 | $i-C_4H_9$ | $C_6H_5$ | $C_6H_5$ | |
| 45 | $CH_3$ | $4-Cl-C_6H_4$ | $C_6H_5$ | 165-167 |
| 46 | $i-C_3H_7$ | $4-Cl-C_6H_4$ | $C_6H_5$ | |
| 47 | $CH_3$ | $4-CH_3-C_6H_4$ | $C_6H_5$ | |
| 48 | $n-C_3H_7$ | $4-CH_3-C_6H_4$ | $C_6H_5$ | |
| 49 | $CH_3$ | $4-CH_3O-C_6H_4$ | $C_6H_5$ | |
| 49 | $CH_3$ | $4-CH_3O-C_6H_4$ | $C_6H_5$ | |
| 50 | $CH_3$ | $3-CF_3O-C_6H_4$ | $C_6H_5$ | |
| 51 | $CH_3$ | $3-CH_3-C_6H_4$ | $C_6H_5$ | |
| 52 | $CH_3$ | $3-CH_3O-C_6H_4$ | $C_6H_5$ | |
| 53 | $CH_3$ | $CH_3$ | $4-CH_3-C_6H_4$ | |
| 54 | $C_2H_5$ | $CH_3$ | $3-CH_5-C_6H_4$ | |
| 55 | $i-C_3H_7$ | $i-C_3H_7$ | $2-CH_3-C_6H_4$ | |
| 56 | $CH_3$ | $CH_3$ | $4-CF_3-C_6H_4$ | 144-145 |
| 57 | $CH_3$ | $i-C_3H_7$ | $4-CF_3-C_6H_4$ | |
| 58 | $i-C_3H_7$ | $i-C_3H_7$ | $4-CF_3-C_6H_4$ | |
| 59 | $CH_3$ | $CH_3$ | $3-CF_3-C_6H_4$ | |
| 60 | $CH_3$ | $n-C_4H_9$ | $3-CF_3-C_6H_4$ | |
| 61 | $i-C_3H_7$ | $i-C_3H_7$ | $3-CF_3-C_6H_4$ | viskos |
| 62 | $CH_3$ | $CH_3$ | $4-CH_3O-C_6H_4$ | |
| 63 | $CH_3$ | $i-C_3H_7$ | $4-CH_3O-C_6H_4$ | |
| 64 | $i-C_3H_7$ | $i-C_3H_7$ | $4-CH_3O-C_6H_4$ | |
| 65 | $CH_3$ | $CH_3$ | $3-CH_3O-C_6H_4$ | |
| 66 | $CH_3$ | $CH_3$ | $4-CF_3O-C_6H_4$ | |
| 67 | $i-C_3H_7$ | $i-C_3H_7$ | $3-CF_3O-C_6H_4$ | |
| 68 | $CH_3$ | $CH_3$ | $3-CH_3-4-Cl-C_6H_3$ | |
| 69 | $C_2H_5$ | $C_2H_5$ | $2-CH_3-4-Cl-C_6H_3$ | |
| 70 | $i-C_3H_7$ | $i-C_3H_7$ | $2-CH_3-4-Cl-C_6H_3$ | 119-121 |
| 71 | $CH_3$ | $CH_3$ | $2-CH_3-5-Cl-C_6H_3$ | |

Die neuen Wirkstoffe zeigen eine starke fungitoxische Wirksamkeit gegen phytopathogene Pilze, insbesondere aus der Klasse der Phycomyceten. Die neuen Verbindungen sind daher beispielsweise geeignet zur Bekämpfung von Phytophthora infestans an Tomaten und Kartoffeln, Phytophthora parasitica an Erdbeeren, Phytophthora cactorum an Äpfeln, Pseudoperonospora cubensis an Gurken, Pseudoperonospora humuli an Hopfen, Peronospora destructor an Zwiebeln, Peronospora sparsa an Rosen, Peronospora tabacina an Tabak, Plasmopara viticola an Reben, Plasmopara halstedii an

Sonnenblumen, Sclerospora macrospora an Mais, Bremia lactucae an Salat, Mucor mucedo an Früchten, Rhizopus nigricans an Rüben. Außerdem besitzen die Wirkstoffe eine vorteilhafte bakterizide Wirksamkeit zum Beispiel gegen Staphylococcus aureus, Escherichia coli, Xanthomonas- und Pseudomonas Arten. Die fungiziden Mittel enthalten 0,1 bis 95 % (Gewichtsprozent) Wirkstoff, vorzugsweise 0,5 bis 90 %. Die Aufwandmengen liegen je nach Art des gewünschten Effektes zwischen 0,1 und 5 kg Wirkstoff je ha. Ein Teil der Wirkstoffe zeigt kurative Eigenschaften, d. h. die Anwendung der Mittel kann noch nach erfolgter Infektion der Pflanzen durch die Krankheitserreger vorgenommen werden, um einen sicheren Bekämpfungserfolg zu erzielen.

Die neuen Wirkstoffe können auch zusammen mit anderen Wirkstoffen, z. B. Herbiziden, Insektiziden, Wachstumsregulatoren und Fungiziden oder auch mit Düngemitteln vermischt und ausgebracht werden. In vielen Fällen erhält man bei der Mischung mit Fungiziden auch eine Vergrößerung des fungiziden Wirkungsspektrums ; bei einer Anzahl dieser Fungizidmischungen treten auch synergistische Effekte auf, d. h. die fungizide Wirksamkeit des Kombinationsproduktes ist größer als die der addierten Wirksamkeiten der Einzelkomponenten. Eine besonders günstige Vergrößerung des Wirkungsspektrums wird beispielsweise mit folgenden Fungiziden erzielt :

Schwefel,
Dithiocarbamate und deren Derivate, wie
Ferridimethyldithiocarbamat
Zinkdimethyldithiocarbamat
Zinkethylenbisdithiocarbamat
Manganethylenbisdithiocarbamat
Mangan-Zink-ethylendiamin-bis-dithiocarbamat
Tetramethylthiuramdisulfide
Ammoniak-Komplex von Zink-(N,N'-ethylen-bis-dithiocarbamat)
Ammoniak-Komplex von Zink-(N,N'-propylen-bis-dithiocarbamat)
Zink-(N,N'-propylen-bis-dithiocarbamat)
N,N'-Propylen-bis-(thiocarbamoyl)-disulfid

Nitroderivate, wie
Dinitro-(1-methylheptyl)-phenylcrotonat
2-sec.-Butyl-4,6-dinitrophenyl-3,3-dimethylacrylat
2-sec.-Butyl-4,6-dinitrophenyl-isopropylcarbonat
5-Nitro-isophthalsäure-di-isopropylester

heterocyclische Strukturen, wie
2-Heptadecyl-2-imidazolin-acetat
2,4-Dichlor-6-(o-chloranilino)-s-triazin
O,O-Diethyl-phthalimidophosphonothioat
5-Amino-1-(bis-(dimethylamino)-phosphinyl)-3-phenyl-1,2,4-triazol
2,3-Dicyano-1,4-dithioanthrachinon
2-Thio-1,3-dithio-(4,5-b)-chinoxalin
1-(Butylcarbamoyl)-2-benzimidazol-carbaminsäuremethylester
2-Methoxycarbonylamino-benzimidazol
2-(Furyl-(2))-benzimidazol
2-(Thiazolyl-(4))-benzimidazol
N-(1,1,2,2-Tetrachlorethylthio)-tetrahydrophthalimid,
N-Trichlormethylthio-tetrahydrophthalimid
N-Trichlormethyl-phthalimid

N-Dichlorfluormethylthio-N',N'-dimethyl-N-phenyl-schwefelsäurediamid
5-Ethoxy-3-trichlormethyl-1,2,3-thiadiazol
2-Rhodanmethylthiobenzthiazol
1,4-Dichlor-2,5-dimethoxybenzol
4-(2-Chlorphenylhydrazono)-3-methyl-5-isoxazolon
Pyridin-2-thio-1-oxid
8-Hydroxychinolin bzw. dessen Kupfersalz
2,3-Dihydro-5-carboxanilido-6-methyl-1,4-oxathiin
2,3-Dihydro-5-carboxanilido-6-methyl-1,4-oxathiin-4,4-dioxid
2-Methyl-5,6-dihydro-4-H-pyran-3-carbonsäure-anilid
2-Methyl-furan-3-carbonsäureanilid
2,5-Dimethyl-furan-3-carbonsäureanilid
2,4,5-Trimethyl-furan-3-carbonsäureanilid
2,5-Dimethyl-furan-3-carbonsäurecyclohexylamid
N-Cyclohexyl-N-methoxy-2,5-dimethyl-furan-3-carbonsäureamid

2-Methyl-benzoesäure-anilid
2-Iod-benzoesäure-anilid
N-Formyl-N-morpholin-2,2,2-trichlorethylacetal
Piperazin-1,4-diylbis-(1-(2,2,2-trichlor-ethyl)-formamid
1-(3,4-Dichloranilino)-1-formylamino-2,2,2-trichlorethan
2,6-Dimethyl-N-tridecyl-morpholin bzw. dessen Salze
2,6-Dimethyl-N-cyclododecyl-morpholin bzw. dessen Salze
N-[3-(p-tert.-Butylphenyl)-2-methylpropyl]-cis-2,6-dimethylmorpholin
N-[3-(p-tert.-Butylphenyl)-2-methylpropyl]-piperidin
1-[2-(2,4-Dichlorphenyl)-4-ethyl-1,3-dioxolan-2-yl-ethyl]-1H-1,2,4-triazol
1-[2-(2,4-Dichlorphenyl)-4-n-propyl-1,3-dioxolan-2-yl-ethyl]-1H-1,2,4-triazol
N-(n-Propyl)-N-(2,4,6-trichlorphenoxyethyl)-N'-imidazol-yl-harnstoff
1-(4-Chlorphenoxy)-3,3-dimethyl-1-(1H-1,2,4-triazol-1-yl)-2-butanon
1-(4-Chlorphenoxy)-3,3-dimethyl-1-(1H-1,2,4-triazol-1-yl)-2-butanol
alpha-(2-Chlorphenyl)-alpha-(4-chlorphenyl)-5-pyrimidin-methanol
5-Butyl-2-dimethylamino-4-hydroxy-6-methyl-pyrimidin
Bis-(p-chlorphenyl)-3-pyridinmethanol
1,2-Bis-(3-ethoxycarbonyl-2-thioureido)-benzol
1,2-Bis-(3-methoxycarbonyl-2-thioureido)-benzol

sowie verschiedene Fungizide, wie
Dodecylguanidinacetat
3-(3-(3,5-Dimethyl-2-oxycyclohexyl)-2-hydroxyethyl)-glutarimid
Hexachlorbenzol
DL-Methyl-N-(2,6-dimethyl-phenyl)-N-furoyl(2)-alaninat
DL-N-(2,6-Dimethyl-phenyl)-N-(2'-methoxyacetyl)-alaninmethylester
N-(2,6-Dimethylphenyl)-N-chloracetyl-D,L-2-aminobutyrolacton
DL-N-(2,6-Dimethylphenyl)-N-(phenylacetyl)-alaninmethylester
5-Methyl-5-vinyl-3-(3,5-dichlorphenyl)-2,4-dioxo-1,3-oxazolidin
3-(3,5-Dichlorphenyl)-5-methyl-5-methoxymethyl-1,3-oxazolidin-2,4-dion
3-(3,5-Dichlorphenyl)-1-isopropylcarbamoylhydantoin
N-(3,5-Dichlorphenyl)-1,2-dimethylcyclopropan-1,2-dicarbonsäureimid

Die neuen Wirkstoffe werden beispielsweise in Form von direkt versprühbaren Lösungen, Pulvern, Suspensionen, auch hochprozentige wäßrige, ölige oder sonstige Suspensionen oder Dispersionen, Emulsionen, Öldispersionen, Pasten, Stäubemitteln, Streumitteln, Granulaten, durch Versprühen, Vernebeln, Verstäuben, Verstreuen, Beizen oder Gießen angewendet. Die Aufwendungsformen richten sich ganz nach den Verwendungszwecken ; sie sollten in jedem Fall möglichst die feinste Verteilung der neuen Wirkstoffe gewährleisten.

Zur Herstellung von direkt versprühbaren Lösungen, Emulsionen, Pasten und Öldispersionen kommen Mineralölfraktionen von mittlerem bis hohem Siedepunkt, wie Kerosin oder Dieselöl, ferner Kohlenteeröle usw., sowie Öle pflanzlichen oder tierischen Ursprungs, aliphatische, cyclische und aromatische Kohlenwasserstoffe, zum Beispiel Benzol, Toluol, Xylol, Paraffin, Tetrahydronaphthalin, alkylierte Naphthaline oder deren Derivate z. B. Methanol, Ethanol, Propanol, Butanol, Chloroform, Tetrachlorkohlenstoff, Cyclohexanol, Cyclohexanon, Chlorbenzol, Isophoron usw., stark polare Lösungsmittel, wie z. B. Dimethylformamid, Dimethylsulfoxid, N-Methylpyrrolidon, Wasser usw. in Betracht.

Wäßrige Anwendungsformen können aus Emulsionskonzentraten, Pasten oder netzbaren Pulvern (Spritzpulvern), Öldispersionen durch Zusatz von Wasser bereitet werden. Zur Herstellung von Emulsionen, Pasten oder Öldispersionen können die Substanzen als solche oder in einem Öl oder Lösungsmittel gelöst, mittels Netz-, Haft-, Dispergier- oder Emulgiermittel in Wasser homogenisiert werden. Es können aber auch aus wirksamer Substanz, Netz-, Haft-, Dispergier- oder Emulgiermittel und eventuell Lösungsmittel oder Öl bestehende Konzentrate hergestellt werden, die zur Verdünnung mit Wasser geeignet sind.

Als oberflächenaktive Stoffe kommen in Betracht : Alkali-, Erdalkali-, Ammoniumsalze von Ligninsulfonsäure, Naphthalinsulfonsäuren, Phenolsulfonsäure, Alkylarylsulfonate, Alkylsulfate, Alkylsulfonate, Alkali- und Erdalkalisalze der Dibutylnaphthalinsulfonsäure, Laurylethersulfat, Fettalkoholsulfate, fettsaure Alkali- und Erdalkalisalze, Salze sulfatierter Hexadecanole, Heptadecanole, Octadecanole, Salze von sulfatiertem Fettalkoholglykolether, Kondensationsprodukte von sulfoniertem Naphthalin und Naphthalinderivaten mit Formaldehyd, Kondensationsprodukte des Naphthalins bzw. der Naphthalinsulfonsäuren mit Phenol und Formaldehyd, Polyoxyethylen-octylphenolether, ethoxyliertes Isooctylphenol-, Octylphenol-, Nonylphenol, Alkylphenolpolyglykolether, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether, ethoxyliertes Polyoxypropylen, Laurylalkoholpolyglykoletheracetal, Sorbitester, Lignin, Sulfitablaugen und Methylcellulose.

Pulver, Streu- und Stäubemittel können durch Mischen oder gemeinsames Vermahlen der wirksamen

Substanzen mit einem festen Trägerstoff hergestellt werden.

Granulate, z. B. Umhüllungs-, Imprägnierungs- und Homogengranulate, können durch Bindung der Wirkstoffe an feste Trägerstoffe hergestellt werden. Feste Trägerstoffe sind z. B. Mineralerden wie Silicagel, Kieselsäuren, Kieselgele, Silikate, Talkum, Kaolin, Attaclay, Kalkstein, Kreide, Talkum, Bolus, Löß, Ton, Dolomit, Diatomeenerde, Calcium- und Magnesiumsulfat, Magnesiumoxid, gemahlene Kunststoffe, Düngemittel, wie z. B. Ammoniumsulfat, Ammoniumphosphat, Ammoniumnitrat, Harnstoffe und pflanzliche Produkte, wie Getreidemehle, Baumrinden-, Holz- und Nußschalenmehle, Cellulosepulver und andere feste Trägerstoffe.

Beispiele für solche Zubereitungen sind :

I. 10 Gewichtsteile der Verbindung Nr. 4 werden in einer Mischung gelöst, die aus 90 Gewichtsteilen Xylol, 6 Gewichtsteilen des Anlagerungsproduktes von 8 bis 10 Mol Ethylenoxid an 1 Mol Ölsäure-N-mono-ethanol-amid, 2 Gewichtsteilen Calciumsalz der Dodecylbenzolsulfonsäure und 2 Gewichtsteilen des Anlagerungsproduktes von 40 Mol Ethylenoxid an 1 Mol Ricinusöl besteht. Durch Eingießen der Mischung in Wasser erhält man eine wäßrige Dispersion.

II. 20 Gewichtsteile der Verbindung Nr. 6 werden in einer Mischung gelöst, die aus 60 Gewichtsteilen Cyclohexanon, 30 Gewichtsteilen Isobutanol, 10 Gewichtsteilen des Anlagerungsproduktes von 40 Mol Ethylenoxid an 1 Mol Ricinusöl besteht. Durch Eingießen und feines Verteilen der Lösung in Wasser erhält man eine waßrige Dispersion.

III. 20 Gewichtsteile der Verbindung Nr. 7 werden in einer Mischung gelöst, die aus 25 Gewichtsteilen Cyclohexanol, 65 Gewichtsteilen einer Mineralölfraktion vom Siedepunkt 210 bis 280 °C und 10 Gewichtsteilen des Anlagerungsproduktes von 40 Mol Ethylenoxid an 1 Mol Ricinusöl besteht. Durch Eingießen und feines Verteilen der Lösung in Wasser erhält man eine wäßrige Dispersion.

IV. 80 Gewichtsteile der Verbindung Nr. 13 werden mit 3 Gewichtsteilen des Natriumsalzes der Diisobutylnaphthalin-alpha-sulfonsäure, 10 Gewichtsteilen des Natriumsalzes einer Ligninsulfonsäure aus einer Sulfitablauge und 7 Gewichtsteilen pulverförmigem Kieselsäuregel gut vermischt und in einer Hammermühle vermahlen. Durch feines Verteilen der Mischung in Wasser erhält man eine Spritzbrühe.

V. 3 Gewichtsteile der Verbindung Nr. 45 werden mit 97 Gewichtsteilen feinteiligem Kaolin innig vermischt. Man erhält auf diese Weise ein Stäubemittel, das 3 Gew.% des Wirkstoffs enthält.

VI. 30 Gewichtsteile der Verbindung Nr. 56 werden mit einer Mischung aus 92 Gewichtsteilen pulverförmigem Kieselsäuregel und 8 Gewichtsteilen Paraffinöl, das auf die Oberfläche dieses Kieselsäuregels gesprüht wurde, innig vermischt. Man erhält auf diese Weise eine Aufbereitung des Wirkstoffs mit guter Haftfähigkeit.

VII. 40 Gewichtsteile der Verbindung Nr. 4 werden mit 10 Teilen Natriumsalz eines Phenolsulfonsäure-harnstoff-formaldehyd-Kondensates, 2 Teilen Kieselgel und 48 Teilen Wasser innig vermischt. Man erhält eine stabile wäßrige Dispersion.

VIII. 20 Teile der Verbindung Nr. 6 werden mit 2 Teilen Calciumsalz der Dodecylbenzolsulfonsäure, 8 Teilen Fettalkoholpolyglykolether, 2 Teilen Natriumsalz eines Phenolsulfonsäure-harnstoff-formaldehyd-Kondensates und 68 Teilen eines paraffinischen Mineralöls innig vermischt. Man erhält eine stabile ölige Dispersion.

Die Wirkstoffe können auch mit gutem Erfolg im Ultra-Low-Volume-Verfahren (ULV) verwendet werden, wobei es möglich ist, Formulierungen mit mehr als 95 Gew.% Wirkstoff oder sogar den Wirkstoff ohne Zusätze auszubringen.

Für die folgenden Versuche wurden als bekannte Vergleichswirkstoffe die folgenden Verbindungen verwendet :

2,6-Dimethyl-4-(N-ethylcarbamoyl)-3,5-dioxo-2H-3,4,5,6-tetrahydro-1,2,6-thiadiazin-1,1-dioxid (Verbindung A) N-Trichlormethylthio-tetrahydrophthalimid (Verbindung B)

## Versuch 1

Wirksamkeit gegen Phytophthora infestans an Tomaten

Blätter von Topfpflanzen der Sorte « Große Fleischtomate » werden mit wäßriger Spritzbrühe, die 80 % Wirkstoff und 20 % Emulgiermittel in der Trockensubstanz enthält, besprüht. Nach dem Antrocknen des Spritzbelages werden die Blätter mit einer Zoosporenaufschwemmung des Pilzes Phytophthora infestans infiziert. Die Pflanzen werden dann in einer wasserdampfgesättigten Kammer bei Temperaturen zwischen 16 und 18 °C aufgestellt. Nach 5 Tagen hat sich die Krankheit auf den unbehandelten, jedoch infizierten Kontrollpflanzen so stark entwickelt, daß die fungizide Wirksamkeit der Substanzen beurteilt werden kann.

Das Ergebnis des Versuches zeigt, daß beispielsweise die Verbindungen Nr. 4, 6, 7, 13, 45 und 56 bei der Anwendung als 0,025 %ige (Gew.%) Spritzbrühe eine bessere fungizide Wirkung (beispielsweise 97 %) zeigen als die bekannten Wirkstoffe A und B (beispielsweise 60 %). Der Wirkstoff A ist stark phytotoxisch und tötet die Tomatenpflanzen ab.

Versuch 2

Wirksamkeit gegen Plasmopara viticola

Blätter von Topfreben der Sorte « Müller-Thurgau » werden mit wäßriger Spritzbrühe, die 80 % Wirkstoff und 20 Emulgiermittel in der Trockensubstanz enthält, besprüht. Um die Wirkungsdauer der Wirkstoffe beurteilen zu können, werden die Pflanzen nach dem Antrocknen des Spritzbelages 10 Tage im Gewächshaus aufgestellt. Erst dann werden die Blätter mit einer Zoosporenaufschwemmung von Plasmopara viticola (Rebenperonospora) infiziert. Danach werden die Reben zunächst für 16 Stunden in einer wasserdampfgesättigten Kammer bei 24 °C und anschließend für 8 Tage in einem Gewächshaus mit Temperaturen zwischen 20 und 30 °C aufgestellt. Nach dieser Zeit werden Pflanzen zur Beschleunigung des Sporangienträgerausbruches abermals für 16 Stunden in der feuchten Kammer aufgestellt. Dann erfolgt die Beurteilung des Ausmaßes des Pilzausbruches auf den Blattunterseiten.

Das Ergebnis des Versuches zeigt, daß beispielsweise die Wirkstoffe Nr. 4, 10 und 56 bei Anwendung als 0,025 %ige (Gew.%) Spritzbrühe eine gute fungizide Wirkung (beispielsweise 97 %) zeigen.

**Patentansprüche** (für die Vertragsstaaten : BE, CH, DE, FR, GB, IT, LI, NL, SE)

1. Thiadiazinon der Formel

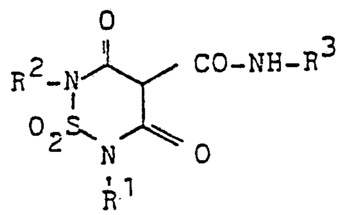

(I)

in der

$R^1$ und $R^2$ gleich oder verschieden sind und Wasserstoff, $C_1$-$C_{10}$-Alkyl, $C_3$-$C_8$-Cycloalkyl, Phenyl oder durch Halogen, Alkoxy, Alkyl oder Halogenalkoxy substituiertes Phenyl,

$R^3$ Phenyl, halogenalkylsubstituiertes, alkylsubstituiertes, alkoxysubstituiertes, halogenalkoxysubstituiertes, oder gleichzeitig durch Alkyl und Halogen substituiertes Phenyl bedeuten.

2. Fungizid, enthaltend ein Thiadiazinon der Formel I gemäß Anspruch 1.

3. Fungizid, enthaltend inerte Zusatzstoffe und ein Thiadiazinon der Formel I gemäß Anspruch 1.

4. Verfahren zur Bekämpfung von Pilzen, dadurch gekennzeichnet, daß man die Pilze oder die vor Pilzbefall zu schützenden Flächen, Pflanzen oder Saatgüter mit einem Thiadiazinon der Formel I gemäß Anspruch 1 behandelt.

5. Verfahren zur Herstellung von Thiadiazinonen der Formel I gemäß Anspruch 1, dadurch gekennzeichnet, daß man ein Thiadiazinon der Formel

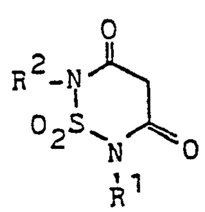

(II)

in der $R^1$ und $R^2$ die vorgenannte Bedeutung besitzen, in einem inerten Lösungsmittel mit einem Isocyanat der Formel

$$R^3—NCO \qquad (III)$$

umsetzt.

6. Thiadiazinon gemäß Anspruch 1, dadurch gekennzeichnet, daß $R^1$ Alkyl mit 1 bis 4 C-Atomen, $R^2$ Alkyl mit 1 bis 4 C-Atomen oder Halogenphenyl, $R^3$ Phenyl oder Trifluormethylphenyl bedeuten.

7. Fungizid gemäß Anspruch 2, dadurch gekennzeichnet, daß $R^1$ Alkyl mit 1 bis 4 C-Atomen, $R^2$ Alkyl mit 1 bis 4 C-Atomen oder Halogenphenyl, $R^3$ Phenyl oder Trifluormethylphenyl bedeuten.

**Patentansprüche** (für den Vertragsstaat AT)

1. Fungizid, enthaltend ein Thiadiazinon der Formel I

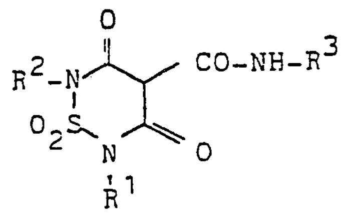

(I)

in der

$R^1$ und $R^2$ gleich oder verschieden sind und Wasserstoff, $C_1$-$C_{10}$-Alkyl, $C_3$-$C_8$-Cycloalkyl, Phenyl oder durch Halogen, Alkoxy, Alkyl oder Halogenalkoxy substituiertes Phenyl,

$R^3$ Phenyl, halogenalkylsubstituiertes, alkylsubstituiertes, alkoxysubstituiertes, halogenalkoxysubstituiertes, oder gleichzeitig durch Alkyl und Halogen substituiertes Phenyl bedeuten.

2. Fungizid, enthaltend inerte Zusatzstoffe und ein Thiadiazinon der Formel I gemäß Anspruch 1.

3. Verfahren zur Bekämpfung von Pilzen, dadurch gekennzeichnet, daß man die Pilze oder die vor Pilzbefall zu schützenden Flächen, Pflanzen oder Saatgüter mit einem Thiadiazinon der Formel I gemäß Anspruch 1 behandelt.

4. Verfahren zur Herstellung von Thiadiazinonen der Formel I gemäß Anspruch 1, dadurch gekennzeichnet, daß man ein Thiadiazinon der Formel

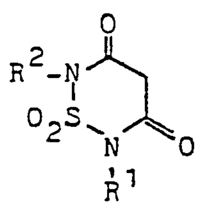

(II)

in der $R^1$ und $R^2$ die vorgenannte Bedeutung besitzen, in einem inerten Lösungsmittel mit einem Isocyanat der Formel

$$R^3—NCO \quad (III)$$

umsetzt.

5. Fungizid gemäß Anspruch 1, dadurch gekennzeichnet, daß $R^1$ Alkyl mit 1 bis 4 C-Atomen, $R^2$ Alkyl mit 1 bis 4 C-Atomen oder Halogenphenyl, $R^3$ Phenyl oder Trifluormethylphenyl bedeuten.

**Claims** (for the Contracting States : BE, CH, DE, FR, GB, IT, LI, NL, SE)

1. A thiadiazinone of the formula

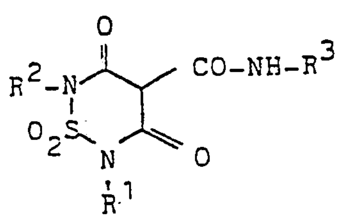

(I)

where

$R^1$ and $R^2$ are identical or different and are each hydrogen, $C_1$-$C_{10}$-alkyl, $C_3$-$C_8$-cycloalkyl, phenyl or halogen-, alkoxy-, alkyl- or halgalkyoxy- substituted phenyl, and

$R^3$ is phenyl or haloalkyl-substituted, alkyl-substituted, alkoxy-substituted or haloalkoxy-substituted or alkyl- and halo-substituted phenyl.

2. A fungicide containing a thiadiazione of the formula I as claimed in claim 1.

3. A fungicide containing inert additives and a thiadiazinone of the formula I as claimed in claim 1.

4. A process for combating fungi, wherein the fungi or the areas, plants or seed to be protected against fungus attack are treated with a thiadiazinone of the formula I as claimed in claim 1.

5. A process for the preparation of a thiadiazinone of the formula I as claimed in claim 1 wherein a thiadiazinone of the formula

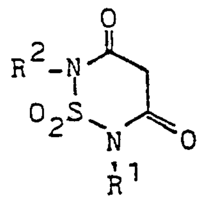

(II)

where $R^1$ and $R^2$ have the above meanings, are reacted with an isocyanate of the formula

$R^3$—NCO (III)

in an inert solvent.

6. A thiadiazinone as claimed in claim 1, where $R^1$ is alkyl of from 1 to 4 carbon atoms, $R^2$ is alkyl of from 1 to 4 carbon atoms or halophenyl, and $R^3$ is phenyl or trifluoromethylphenyl.

7. A fungicide as claimed in claim 2, where $R^1$ is alkyl of from 1 to 4 carbon atoms, $R^2$ is alkyl of from 1 to 4 carbon atoms or halophenyl, and $R^3$ is phenyl or trifluoromethylphenyl.

**Claims** (for the Contracting State AT)

1. A fungicide containing a thiadiazinone of the formula I

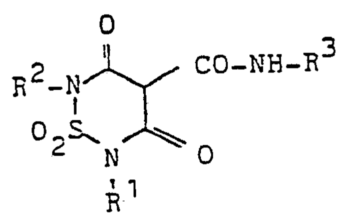

(I)

where

$R^1$ and $R^2$ are identical or different and are each hydrogen, $C_1$-$C_{10}$-alkyl, $C_3$-$C_8$-cycloalkyl, phenyl or halogen-, alkoxy-, alkyl- or haloalkoxy- substituted phenyl and

$R^3$ is phenyl or haloalkyl-substituted, alkyl-substituted, alkoxy-substituted or haloalkoxy-substituted or alkyl- and halo-substituted phenyl.

2. A fungicide containing inert additives and a thiadiazinone of the formula I as claimed in claim 1.

3. A process for combating fungi, wherein the fungi or the areas, plants or seed to be protected against fungus attack are treated with a thiadiazinone of the formula I as claimed in claim 1.

4. A process for the preparation of a thiadiazinone of the formula I as claimed in claim 1, wherein thiadiazinone of the formula

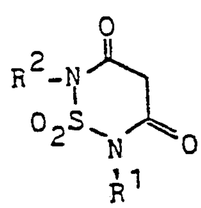

(II)

where $R^1$ and $R^2$ have the above meanings, are reacted with an isocyanate of the formula

$R^3$—NCO (III)

in an inert solvent.

5. A fungicide as claimed in claim 1, where $R^1$ is alkyl of from 1 to 4 carbon atoms, $R^2$ is alkyl of from 1 to 4 carbon atoms or halophenyl, and $R^3$ is phenyl or trifluoromethylphenyl.

**Revendications** (pour les Etats contractants : BE, CH, DE, FR, GB, IT, LI, NL, SE)

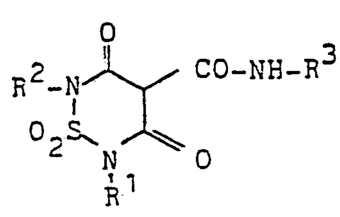

(I)

dans laquelle

$R^1$ et $R^2$ sont identiques ou différents et représentent hydrogène, alkyle en $C_1$-$C_{10}$, cycloalkyle en $C_3$-$C_8$, phényle ou phényle substitué par halogène, alcoxy, alkyle ou halogène-alcoxy,

$R^3$ représente phényle ou phényle substitué par halogène-alkyle, alkyle, alcoxy, halogène-alcoxy ou à la fois par alkyle et halogène.

2. Fongicide contenant une thiadiazinone de formule I, selon la revendication 1.

3. Fongicide contenant des additifs inertes et une thiadiazinone de formule I, selon la revendication 1.

4. Procédé de lutte contre les champignons, caractérisé par le fait que l'on traite les champignons ou les surfaces, plantes ou semences à protéger contre l'attaque des champignons, par une thiadiazinone de formule I, selon la revendication 1.

5. Procédé de préparation de thiadiazinones de formule I, selon la revendication 1, caractérisé par le fait que l'on fait réagir, dans un solvant inerte, une thiadiazinone de formule

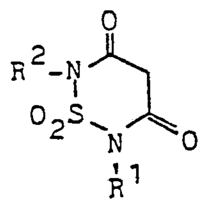

(II)

dans laquelle $R^1$ et $R^2$ ont les significations données plus haut, avec un isocyanate de formule

$$R^3\text{—NCO} \qquad \text{(III)}$$

6. Thiadiazinone selon la revendication 1, caractérisé par le fait que $R^1$ représente alkyle à 1 à 4 atomes C, $R^2$ représente alkyle à 1 à 4 atomes C ou halogène-phényle, $R^3$ étant phényle ou trifluorométhylphényle.

7. Fongicide selon la revendication 2, caractérisé par le fait que $R^1$ représente alkyle à 1 à 4 atomes C, $R^2$ représente alkyle à 1 à 4 atomes C ou halogène-phényle, $R^3$ étant phényle ou trifluorométhylphényle.

**Revendications** (pour l'Etat contractant AT)

1. Fongicide contenant une thiadiazinone de formule I

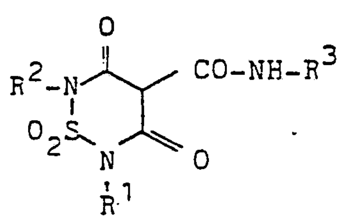

(I)

dans laquelle

$R^1$ et $R^2$ sont identiques ou différents et représentent hydrogène, alkyle en $C_1$-$C_{10}$, cycloalkyle en $C_3$-$C_8$, phényle ou phényle substitué par halogène, alcoxy, alkyle ou halogène-alcoxy,

$R^3$ représente phényle ou phényle substitué par halogène-alkyle, alkyle, alcoxy, halogène-alcoxy ou à la fois par alkyle et halogène.

2. Fongicide contenant des additifs inertes et une thiadiazinone de formule I, selon la revendication 1.

3. Procédé de lutte contre les champignons, caractérisé par le fait que l'on traite les champignons ou les surfaces, plantes ou semences à protéger contre l'attaque de champignons, par une thiadiazinone de formule I, selon la revendication 1.

4. Procédé de préparation de thiadiazinones de formule I, selon la revendication 1, caractérisé par le fait que l'on fait réagir, dans un solvant inerte, une thiadiazinone de formule

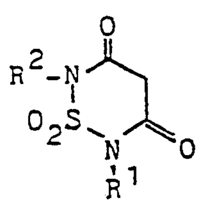

(II)

dans laquelle $R^1$ et $R^2$ ont les significations données plus haut, avec un isocyanate de formule

$$R^3\text{—NCO} \qquad (III)$$

5. Fongicide selon la revendication 1, caractérisé par le fait que $R^1$ représente alkyle à 1 à 4 atomes C, $R^2$ représente alkyle à 1 à 4 atomes C ou halogène-phényle, $R^3$ étant phényle ou trifluorométhylphényle.